(19) Europäisches Patentamt
European Patent Office
Office européen des brevets

(11) **EP 1 351 603 B1**

(12) **FASCICULE DE BREVET EUROPEEN**

(45) Date de publication et mention
de la délivrance du brevet:
**11.05.2011   Bulletin 2011/19**

(51) Int Cl.:
***A61B 5/053*** *(2006.01)*        *A61B 5/05* *(2006.01)*

(21) Numéro de dépôt: **02785540.2**

(22) Date de dépôt: **11.10.2002**

(86) Numéro de dépôt international:
**PCT/FR2002/003477**

(87) Numéro de publication internationale:
**WO 2003/030735 (17.04.2003 Gazette 2003/16)**

(54) **Pèse-personne comprenant un appareil de mesure de la composition corporelle**

Personenwaage beinhaltend ein Gerät zur Messung der Körperzusammenstellung

Body weight scale comprising an apparatus for measuring body composition

(84) Etats contractants désignés:
**AT BE BG CH CY CZ DE DK EE ES FI FR GB GR
IE IT LI LU MC NL PT SE TR**

(30) Priorité:  **12.10.2001  FR 0113193**

(43) Date de publication de la demande:
**15.10.2003   Bulletin 2003/42**

(73) Titulaire: **SEB S.A.
69130 Ecully (FR)**

(72) Inventeurs:
• **SIMOND, Bénédicte
F-74150 Bloye (FR)**
• **DUBORPER, Alain
F-74150 Sales (FR)**
• **SARRAZIN, Michel
F-74150 Massingy (FR)**

(74) Mandataire: **Kiehl, Hubert
SEB Développement
Les 4 M-Chemin du Petit Bois
B.P. 172
69134 Ecully Cedex (FR)**

(56) Documents cités:
**EP-A- 0 940 120        EP-A2- 0 869 360
WO-A-96/08198        WO-A1-98/37829
JP-A- 10 005 187        US-A- 6 058 325**

• **ITKIS M. ET AL: 'The square-wave approach to
impedance measurement of brain tissue water
dynamics' JOURNAL OF NEUROSCIENCE
METHODS vol. 59, no. 2, Juillet 1995,
NETHERLANDS, pages 237 - 244**

## Description

La présente invention concerne un appareil de mesure de la composition corporelle comportant un circuit électrique de mesure associé à une unité de calcul de l'impédance bioélectrique d'une personne.

La mesure de la composition corporelle donne des indications sur l'état de santé d'une personne. Plus particulièrement, la connaissance de la quantité de masse grasse de l'organisme et de son évolution dans le temps constitue une aide efficace lors d'un régime. Parmi les méthodes de mesure de la masse grasse, on relève la mesure de l'impédance du corps humain corrélée avec la quantité d'eau dans le corps : le corps humain est composé de masse maigre, constituée par les muscles, les os, les organes, les tissus, de masse grasse. L'eau constitue environ 70% de la masse maigre, alors que la masse grasse n'en contient presque pas. Par conséquent, en mesurant l'impédance électrique du corps humain et le poids total d'une personne, on peut calculer, moyennant quelques paramètres d'ajustement, la masse grasse et la masse maigre de la personne.

La mesure de l'impédance bioélectrique est basée sur la conductibilité des tissus lors du passage d'un courant alternatif bref de haute fréquence et faible intensité. Le Dr. Boulier - Université Paris V - Paris - France est à l'origine d'un concept utilisant la seule mesure d'un segment corporel (tronc ou membres inférieurs) parcouru par un signal électrique de fréquence très élevée (supérieur à 100kHz) pour établir la composition corporelle globale permettant ainsi l'utilisation d'un pèse-personne comme support de mesure. Il a établi la première validation scientifique de ce concept en 1994 (Thèse de Doctorat de Biologie Humaine - Université René Descartes - Paris V-1994 p85-86).

Un tel appareil de mesure est décrit dans le document FR-A-2698779 où la valeur de l'impédance du corps est obtenue en utilisant l'association d'un plateau de pèse-personne et d'une plaque munie de quatre électrodes, dont deux sont associées à un circuit comportant un générateur de courant et les deux autres sont reliées à un appareil de mesure. Ainsi, on calcule la valeur de l'impédance de la partie du corps mesurée en fonction de la tension mesurée, en connaissant le courant constant appliqué, et ensuite on calcule la masse maigre ou la masse grasse en appliquant des facteurs d'ajustement en fonction entre autres de la taille, de l'âge et en intégrant le poids de la personne. Cet appareil est d'une utilisation simple, mais les mesures ne sont pas complètement fiables, celles-ci pouvant être faussées par exemple dans le cas des personnes présentant des oedèmes ou des problèmes de rétention d'eau.

En étudiant le comportement électrique du corps humain, on constate qu'il peut être assimilé à une suspension de cellules dans une solution électrolytique conductrice et homogène. Les milieux conducteurs intracellulaire et extracellulaire sont séparés par une membrane lipoprotéique constituant la membrane cellulaire. Le modèle de Fricke assimile le comportement électrique du corps humain à un circuit électrique composé d'une capacité C représentant la membrane cellulaire reliée en série avec une résistance intracellulaire $R_i$ représentant la résistance électrique du milieu intracellulaire, les deux étant reliées en parallèle avec la résistance extracellulaire $R_e$. Afin d'associer d'une manière fiable les mesures d'impédance effectuées à la présence et la localisation de l'eau dans l'organisme, notamment dans le milieu intracellulaire et dans le milieu extracellulaire, il faut connaître leurs résistances respectives $R_i$ et $R_e$.

Le document WO 98/37829 décrit un dispositif de mesure et de localisation de l'apex d'un canal radiculaire d'une dent au moyen des mesures de tension électriques entre une lime formant électrode insérée dans le canal radiculaire et une seconde électrode disposée dans la bouche d'un patient. Le signal appliqué est carré et le dispositif mesure trois valeurs de tension sur une alternance, mesures qui sont utilisées ensuite par des moyens de calcul du dispositif pour déterminer la localisation de l'apex. Toutefois, ce dispositif trouve ses limites lorsqu'il fait appel à des mesures invasives et de surcroît, ses moyens de calcul ne sont nullement prévus pour déterminer la composition corporelle d'un individu. Une solution a été proposée dans le document EP-A-0 940 120 au nom de la demanderesse. Ce document décrit un appareil de mesure de la composition corporelle utilisant une source de courant qui applique un signal de courant de forme carrée et de durée réglable entre les électrodes d'excitation de l'appareil. Des mesures de tension sont effectuées ensuite entre les électrodes de mesure pour déterminer l'impédance globale de la partie du corps mesurée et la résistance extracellulaire et pour en déduire par la suite la résistance intracellulaire. Fonctionnant à satisfaction, cet appareil s'avère d'une réalisation délicate et coûteuse. Ainsi, la méthode de calcul utilisée exige que les mesures soient effectuées à des instants extrêmement précis et, en même temps, que les signaux en courant appliqués soient très précis et qu'il aient un rapport cyclique parfaitement équilibré. Or, l'utilisation d'un tel générateur de courant nécessite la mise en oeuvre de transistors qui sont très sensibles aux conditions de fonctionnement de l'appareil, par exemple aux variations de température, ce qui se traduit sur le signal par une valeur du courant généré qui est influencée par la température ambiante.

Le but de la présente invention est de remédier aux inconvénients précités et de proposer un appareil de mesure de la composition corporelle apte à effectuer des mesures préciser, sans qu'elles soient influencées par les conditions ambiantes lors de son fonctionnement.

Un autre but de l'invention est un appareil de mesure de la composition corporelle qui soit facile à mettre en oeuvre, tout en étant fiable en fonctionnement.

Un but supplémentaire de l'invention est un appareil de mesure de la composition corporelle qui soit d'une construction simplifiée, qui puisse être fabriqué en grande série pour un moindre coût.

Ces buts sont atteints avec un appareil de mesure de la composition corporelle d'un individu comportant un premier module électronique destiné à mesurer une impédance bioélectrique aux bornes d'électrodes de mesure de l'appareil et au moins une source d'énergie électrique destinée à générer un signal électrique variable qui traverse le corps ou une partie du corps de l'individu lorsque ce dernier est relié à l'appareil de mesure moyennant des électrodes d'excitation, du fait que ladite source d'énergie est un générateur de tension destiné à fournir un signal en tension de forme rectangulaire qui est appliqué aux bornes des électrodes d'excitation de l'appareil, que ledit premier module électronique mesure des tensions aux bornes des électrodes de mesure de l'appareil et que des moyens de calcul de l'appareil déterminent la résistance équivalente en fonction de résistances extracellulaire et intracellulaire du corps ou de la partie mesurée du corps de l'individu.

Ainsi, l'utilisation d'un générateur de tension en tant que source d'énergie présente l'avantage de pouvoir fournir en permanence un signal constant, do valeur égale ou inférieure à celle de sa tension d'alimentation. Par exemple, l'appareil peut faire appel à l'utilisation d'un microprocesseur qui génère directement une tension égale à sa tension d'alimentation, moyennant un régulateur électronique de tension qui permet de conserver la tension d'alimentation de l'ensemble de montage électronique constante quel que soit le niveau de tension des piles de l'appareil. De ce fait, de par son architecture interne, le microprocesseur seul est capable de générer un signal rectangulaire en tension, dont les seuils de tension ont des valeurs aussi précises que la précision du régulateur de tension. Ce microprocesseur peut avantageusement être le même que celui utilisé pour réaliser les calculs permettant de déterminer la résistance extracellulaire et intracellulaire de l'organisme, tel qu'il sera expliqué par la suite.

Tel n'était pas le cas avec un générateur de courant selon le document FR-A-2775581 de l'état de la technique où les divers composants utilisés étant sensibles aux paramètres ambiants, l'appareil exigeait l'introduction dans le montage électronique d'un système de compensation en température coûteux et difficile à mettre au point avec précision.

Or, dans l'appareil de l'invention, le signal électrique qui traverse le corps de l'individu est produit par un générateur de tension, générateur qui fournit ainsi un signal stable, de bonne qualité pour un moindre coût et une mise en oeuvré grandement facilitée.

Par ailleurs, le fait d'utiliser, d'une part, un signal de forme rectangulaire et, d'autre part, en tension permet d'éviter une mesure complexe et moins précise de déphasage, comme dans le cas des appareils de mesure de l'impédance bioélectrique de l'état de la technique qui utilisent un signal sinusoïdal et un traitement multifréquence et, simultanément, permet de s'affranchir de l'effet capacitif des tissus du corps et de mesurer les valeurs des résistances intracellulaire et extracellulaire de manière plus simple, sans faire appel à des mesures à des instants extrêmement précis et sans utiliser des signaux ayant un rapport cyclique parfaitement équilibré, comme dans le cas du brevet susmentionné au nom de la demanderesse. Avec un signal en tension on arrive donc à mesurer les résistances intracellulaire et extracellulaire de manière simplifiée, à partir d'un signal unique et invariant au cours d'une même mesure. Avantageusement, l'appareil de l'invention comprend des moyens permettant d'effectuer des mesures de tension à au moins deux instants prédéterminés de la durée dudit signal afin de déterminer directement la résistance équivalente $R_{eq}$ du corps ou de la partie mesurée du corps de l'individu à ces deux instants et d'en déduire sa résistance extracellulaire $R_e$ et sa résistance intracellulaire $R_i$.

Ainsi, avec seulement deux mesures on peut déduire de la résistance équivalente $R_{eq}$ à ces deux instants, en effectuant les calculs nécessaires, les valeurs des résistances intracellulaire et extracellulaire, valeurs qui sont essentielles dans le calcul de la composition corporelle de l'individu, notamment dans la détermination de la répartition de l'eau dans les tissus.

De préférence, l'appareil de l'invention comprend des moyens permettant d'effectuer une première mesure de tension, à la fin d'un palier dudit signal de forme rectangulaire, pour déterminer la résistance équivalente $R_{eq}$, égale à la résistance extracellulaire $R_e$, ainsi qu'une deuxième mesure de tension, à la fin d'un front montant dudit signal pour déterminer la résistance équivalente $R_{eq}$ égale à $R_e$ en parallèle avec $R_i$, et déterminer, sur la base de la première mesure, la résistance intracellulaire $R_i$ du corps ou de la partie mesurée du corps de l'individu.

Un palier dudit signal peut être considéré en théorie comme un signal continu et en pratique à très basse fréquence (inférieure à 10 kHz) alors qu'un front de transition, montant ou descendant, dudit signal peut être considéré en théorie comme un signal de fréquence infinie et en pratique supérieure à 100 kHz. Ainsi, en effectuant une mesure en fin de palier dudit signal, la capacité membranaire C de la partie mesurée du corps de l'individu est considérée comme un circuit ouvert, alors qu'à la fin d'un front montant, la capacité membranaire C est comme un court-circuit, ce qui permet de déterminer les valeurs desdites résistances sans prendre en compte les valeurs de la capacité membranaire C.

De préférence, ledit générateur de tension est destiné à fournir un signal en tension qui présente une forme d'onde dont la période, l'amplitude et le rapport cyclique sont réglables.

Le réglage de la période et du rapport cyclique dudit signal permet de mesurer au mieux la résistance extracellulaire du corps ou de la partie du corps mesurée en adaptant la durée d'un palier du signal à la fin duquel on effectue la mesure pour réaliser la charge complète de la capacité membranaire C afin de pouvoir la considérer comme un circuit ouvert.

Utilement, ledit générateur de tension est destiné à fournir un signal en tension qui est un signal basse tension d'amplitude comprise entre 3 et 6V. Le courant électrique délivré n'est pas supérieur à 800μA.

Une telle tension protège l'individu lors de l'utilisation de l'appareil, tout en assurant un signal suffisamment important pour effectuer des mesures précises.

De préférence, l'appareil de l'invention comporte un deuxième module électronique destiné à mesurer le poids de l'individu.

Ainsi, le deuxième module peut mesurer le poids de l'individu qui est directement fourni à l'appareil, ce qui évite d'entrer manuellement la donnée du poids en vue du calcul de sa composition corporelle.

Utilement, le premier module électronique et le deuxième module électronique sont reliés à une unité de calcul apte à déterminer la composition corporelle de l'individu. Cette unité de calcul peut avantageusement être un microprocesseur.

Les données saisies lors des mesures effectuées avec les deux modules électroniques de l'appareil sont transmises à une unité de calcul, notamment un microprocesseur qui stocke en mémoire les équations de calcul et qui produit ensuite les informations sur la composition corporelle de l'individu.

Avantageusement, l'invention comporte un moyen d'affichage des valeurs mesurées et des valeurs calculées.

Ainsi, l'utilisateur de l'appareil peut être informé à tout moment et en temps réel des valeurs mesurées et/ou calculées par l'appareil.

De préférence, l'appareil de l'invention comporte au moins deux électrodes d'excitation destinées à appliquer ledit signal électrique entre un premier point et un deuxième point sur le corps de l'individu et au moins deux électrodes de mesure aux bornes desquelles est mesurée une tension.

On peut en principe réaliser une mesure d'impédance bioélectrique en utilisant seulement deux électrodes appliquées sur le corps de l'individu et reliées au circuit de mesure. Cependant, l'utilisation de quatre électrodes, dont deux d'application du signal et deux de mesure, permet de passer outre la barrière cutanée au point de contact du corps de l'individu avec les électrodes et d'effectuer ainsi des mesures directement aux bornes du circuit électrique représentant la partie mesurée du corps de l'individu.

Les caractéristiques de l'invention peuvent avantageusement être retrouvées dans un pèse-personne comportant un premier module électronique destiné à mesurer une impédance bioélectrique et au moins une source d'énergie électrique destinée à délivrer un signal électrique variable qui traverse le corps ou une partie du corps de l'individu lorsque ce dernier est relié à un appareil de mesure, du fait que ladite source d'énergie est un générateur de tension destiné à fournir un signal en tension de forme rectangulaire.

L'invention concerne également un procédé de mesure de l'impédance bioélectrique du corps ou d'une partie du corps d'un individu, où l'on applique un signal rectangulaire en tension entre deux électrodes d'excitation reliées à l'individu, l'on mesure des tensions entre deux électrodes de mesure reliées également à l'individu à au moins deux instants prédéterminés de la durée dudit signal rectangulaire permettant de déterminer directement la résistance équivalente $R_{eq}$ à ces deux instants du corps ou de la partie mesurée du corps de l'individu traversée par le signal électrique et d'en déduire sa résistance extracellulaire $R_e$ et sa résistance intracellulaire $R_i$ et l'on détermine la composition corporelle de l'individu en utilisant les valeurs mesurées et une unité de calcul.

De préférence, selon le procédé de l'invention, on effectue une première mesure de tension, à la fin d'un palier dudit signal de forme rectangulaire, pour déterminer la résistance équivalente $R_{eq}$ égale à la résistance extracellulaire $R_e$, ainsi qu'une deuxième mesure de tension, à la fin d'un front montant dudit signal pour déterminer la résistance équivalente $R_{eq}$ égale à $R_e$ en parallèle avec $R_i$ et déterminer, sur la base de la première mesure, la résistance intracellulaire $R_i$ du corps ou de la partie mesurée du corps de l'individu.

D'autres caractéristiques et avantages de l'invention apparaîtront plus clairement à la lumière de la description et des dessins qui suivent, illustrant, à titre d'exemples non limitatifs, des modes de mise en oeuvre de l'invention. Ainsi, référence est faite aux figures 1 à 6, où :

- la figure 1 représente un schéma de principe de mesure de l'impédance bioélectrique utilisant quatre électrodes, selon l'état antérieur de la technique;
- la figure 2 représente un schéma bloc d'un appareil de mesure de la composition corporelle selon l'invention;
- la figure 3 représente une vue de dessus de l'appareil de l'invention;
- la figure 4 représente le schéma électrique de mesure des paramètres de la composition corporelle en utilisant l'appareil de l'invention;
- la figure 5 représente la forme d'onde du signal électrique généré selon un premier mode de réalisation de l'invention;
- la figure 6 représente la forme d'onde du signal électrique généré selon un deuxième mode de réalisation de l'invention.

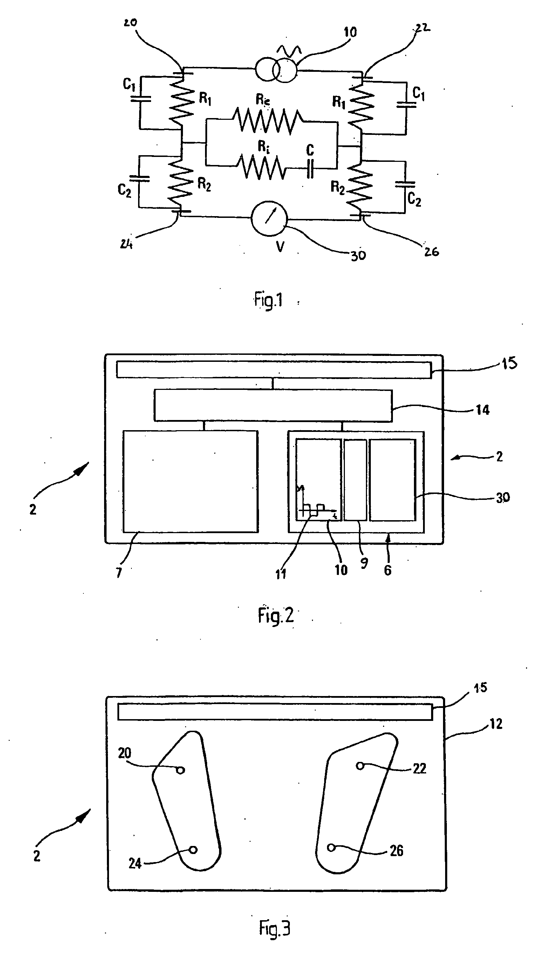

L'appareil de mesure de la composition corporelle 2 d'un individu, humain ou animal, selon l'invention se présente, tel que représenté à la figure 3, sous forme d'un plateau 12 muni de quatre électrodes dont deux d'excitation ou d'application du signal 20,22 entre deux points du corps de l'individu, et deux autres électrodes de mesure 24,26.

Les électrodes peuvent être agencées de manière à venir en contact avec les pieds de l'individu lorsqu'il se tient debout sur le plateau horizontal 12 en vue d'une prise de mesure. Les électrodes 20,22,24,26 sont réalisées en acier inoxydable ou en tout autre matériau électriquement conducteur, alors que le plateau 12 est réalisé en une matière isolante, par exemple une matière plastique. On peut également disposer sur le plateau 12 un dispositif d'affichage 15 des valeurs mesurées et/ou calculées par l'appareil 2.

Avantageusement, le plateau 12 est associé au plateau d'application de charge d'un pèse-personne, dans quel cas les informations lues par ce dernier sont transmises directement au circuit électronique de l'appareil.

Tel que visible à la figure 2, le circuit électronique de l'appareil 2 de l'invention comporte un premier module électronique 6 destiné à mesurer l'impédance bioélectrique d'un individu. Ce module est constitué d'une source d'énergie électrique, notamment un générateur de tension 10, permettant d'envoyer un signal électrique 11 traversant le corps ou une partie du corps de l'individu lorsque ce dernier est relié aux électrodes de l'appareil, d'une unité de mesure 30 et d'une unité de traitement des signaux 9. L'unité de mesure 30 récupère le signal électrique 11 après son passage dans le corps de l'individu entre les électrodes de mesure 24,26.

L'appareil de l'invention comporte un deuxième module électronique 7 destiné à mesurer le poids de l'individu. Le premier module électronique 6 et le deuxième module électronique 7 sont reliés à une unité de calcul 14, notamment un microprocesseur ou un microcontrôleur adaptés au traitement à effectuer. L'unité de calcul 14 est reliée à un dispositif d'affichage 15 des valeurs mesurées et/ou des valeurs calculées par l'appareil 2.

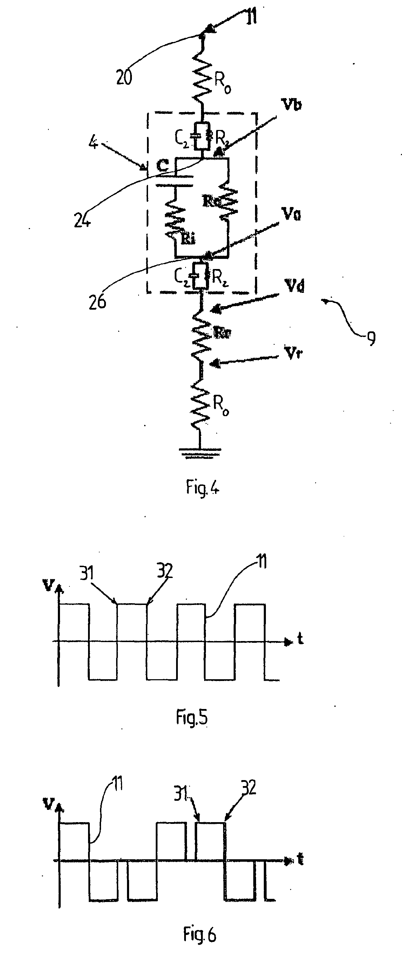

La figure 4 illustre le schéma électrique de l'unité de traitement des signaux 9 selon l'invention du premier module électronique 6 de l'appareil 2. Sur le schéma on retrouve le signal électrique 11 appliqué à l'électrode d'excitation 20. Par la référence 4 on a désigné le modèle électrique du corps ou de la partie mesurée du corps de l'individu, notamment du corps humain, selon le modèle de Fricke, où $R_i$ et $R_e$ sont les résistances intracellulaire et extracellulaire et C représente la capacité membranaire du corps humain ou de la partie mesurée du corps humain. Les références 24 et 26 désignent les électrodes de mesure et $R_2$ et $C_2$ sont les résistances et capacités de contact cutanées de la partie du corps reliée aux électrodes, notamment des pieds. Par $R_0$ on a représenté une résistance de connexion.

Avec le schéma de la figure 4 on se propose de mesurer les tensions $V_b$ et $V_c$ sur chacune des électrodes de mesure 24,26 de l'appareil, ainsi que les tensions $V_d$ et $V_r$ aux bornes d'une résistance de référence $R_r$ calibrée, ayant une bonne précision.

Ainsi, en mesurant les tensions $V_d$ et $V_r$ on obtient la différence de potentiel :

$$V_d - V_r = R_r \cdot i$$

d'où l'on peut déduire l'intensité du courant électrique du circuit représenté :

$$i = (V_d - V_r) / R_r$$

De la même manière, en mesurant les tensions $V_b$ et $V_c$ on obtient la différence de potentiel :

$$V_b - V_c = R_{eq} \cdot i$$

En remplaçant dans cette formule i calculé précédemment, on obtient :

$$R_{eq} = (V_b - V_c) / i = R_r \cdot (V_b - V_c) / (V_d - V_r) \qquad\qquad (1)$$

où, dans cette relation $R_{eq}$ représente la résistance équivalente des membres inférieurs du corps de l'individu aux instants de mesure des tensions $V_b$, $V_c$, $V_d$ et $V_r$.

Le signal utilisé présente une tension de forme d'onde rectangulaire ou carrée dont la durée, l'amplitude et le rapport cyclique sont réglables. La figure 5 montre le signal électrique 11 utilisé dans la mesure de la composition corporelle selon un premier mode de réalisation de l'invention.

La particularité du signal électrique généré 11 est de présenter au point 31, à front montant, un signal de fréquence infinie et au point 32, en fin de palier, un signal très basse fréquence. On connaît par ailleurs que la capacité membranaire C du circuit représenté se comporte comme un court-circuit en haute fréquence, alors qu'en basse fréquence elle se comporte comme un circuit ouvert.

Ainsi, on effectue des mesures des quatre tensions $V_b$, $V_c$, $V_d$ et $V_r$ aux instants 31 et 32, ce qui permet de déduire :

- au point 31 : $1/R_{eq} = 1/R_e + 1/R_i$, avec C en court-circuit, $R_e$ et $R_i$ en parallèle, d'où

$$R_{eq} = (R_e \cdot R_i) / (R_e + R_i) \qquad\qquad (2)$$

- au point 32 : $R_{eq} = R_e$ , avec C en circuit ouvert.

Par conséquent, une mesure des tensions $V_b$, $V_c$, $V_d$ et $V_r$ au point 32 permet de déduire avec la relation (1) la valeur de $R_e$ :

$$R_e = R_{eq} = R_r \cdot (V_b - V_c) / (V_d - V_r) \qquad\qquad (3)$$

Une deuxième mesure des tensions $V_b$, $V_c$, $V_d$ et $V_r$ au point 31 permet de déduire par la suite avec la relation (2) la valeur de $R_i$ :

$$R_i = R_e \cdot R_{eq} / (R_e - R_{eq})$$

où $R_{eq}$ se calcule avec la relation (1) en introduisant les valeurs mesurées des tensions au point 31 et la valeur de $R_e$ a été déterminée au point 32 avec la relation (3).

En conclusion, avec le même signal on peut déterminer $R_{eq}$, ainsi que la résistance intracellulaire $R_i$ et la résistance extracellulaire $R_e$. Les valeurs $R_i$ et $R_e$ sont essentielles pour le calcul de la masse grasse et de la masse maigre. Les calculs sont effectués par l'unité de calcul 14 sur la base des formules mathématiques stockées dans sa mémoire. Ces formules intègrent d'autres paramètres tels : le poids, la taille, l'âge, etc. Ainsi, une mesure précise de la résistance totale représentée par $R_i$ en parallèle avec $R_e$ permet de déterminer la quantité de masse grasse et de masse maigre, et les mesures précises de $R_i$ et $R_e$ permettent d'observer la répartition de l'eau et de détecter, par exemple la présence d'un oedème, ou des problèmes de rétention d'eau, ou encore ces mesures permettent de déterminer la quantité de masse grasse et de masse maigre chez les enfants qui présentent une variation brusque dans le temps de la quantité d'eau contenue dans les tissus musculaires.

La figure 6 présente un deuxième mode de réalisation de l'invention comportant un signal électrique de tension 11 ayant une forme d'onde rectangulaire ou carrée où le signal 11 est annulé au milieu de chaque alternance. Ainsi, les résistances $R_i$ et $R_e$ sont calculées en effectuant des mesures des tensions aux points 31,32 comme précédemment décrit. Ce mode de réalisation permet d'éviter la charge progressive des capacités représentant les parois membranaires.

Le premier module électronique 6, l'unité de traitement de signal, l'unité de mesure 30 ensemble avec l'unité de calcul 14 et le dispositif d'affichage 15 peuvent constituer un appareil indépendant de mesure de la composition corporelle ou ils peuvent être intégrés à d'autres appareils par exemple à un pèse-personne, ou à un appareil de massage, de fitness, etc.

D'autres variantes et modes de réalisation de l'invention peuvent être envisagées sans sortir du cadre de ses revendications.

**Revendications**

1. Pèse-personne comprenant un appareil de mesure de la composition corporelle d'un individu comportant un premier module électronique (6) destiné à mesurer une impédance bioélectrique et au moins une source d'énergie électrique destinée à délivrer un signal électrique variable qui traverse le corps ou une partie du corps de l'individu lorsque ce dernier est relié à l'appareil de mesure moyennant des électrodes d'excitation, **caractérisé en ce que** ladite source d'énergie est un générateur de tension (10) destiné à fournir un signal en tension (11) de forme rectangulaire qui est appliqué aux bornes des électrodes d'excitation de l'appareil, que ledit premier module électronique (6) mesure des tensions aux bornes des électrodes de mesure de l'appareil.

2.  Pèse-personne selon la revendication 1, **caractérisé en ce que** des moyens de calcul de l'appareil déterminent la résistance équivalente en fonction de résistances extracellulaire et intracellulaire du corps ou de la partie mesurée du corps de l'individu.

3.  Pèse-personne selon la revendication 2, **caractérisé en ce que** ledit premier module (6) comprend des moyens de mesure des tensions $V_b$ et $V_c$ aux bornes des électrodes de mesure de l'appareil et des tensions $V_r$ et $V_d$ aux bornes d'une résistance de référence $R_r$ et qu'il comprend des moyens de calcul de la résistance équivalente du corps ou de la partie mesurée du corps de l'individu selon la formule :

$$R_{eq} = R_r \cdot (V_b - V_c) / (V_d - V_r)$$

4.  Pèse-personne selon la revendication 3, **caractérisé en ce que** lesdits moyens de mesure effectuent des mesures de tension à au moins deux instants prédéterminés de la durée dudit signal (11) et des moyens de calcul aptes à déterminer directement la résistance équivalente $R_{eq}$ du corps ou de la partie mesurée du corps de l'individu à ces deux instants et d'en déduire sa résistance extracellulaire $R_e$ et sa résistance intracellulaire $R_i$.

5.  Pèse-personne selon la revendication 4, **caractérisé en ce qu'**il comprend des moyens permettant d'effectuer une première mesure de tension, à la fin d'un palier (32) dudit signal (11) de forme rectangulaire, pour déterminer la résistance équivalente $R_{eq}$ égale à la résistance extracellulaire $R_e$, ainsi qu'une deuxième mesure de tension, à la fin d'un front montant (31) dudit signal (11) pour déterminer la résistance équivalente $R_{eq}$ égale à $R_e$ en parallèle avec $R_i$ et déterminer, sur la base de la première mesure, la résistance intracellulaire $R_i$ du corps ou de la partie mesurée du corps de l'individu.

6.  Pèse-personne selon l'une des revendications précédentes, **caractérisé en ce que** ledit générateur de tension (10) est destiné à fournir un signal en tension (11) qui présente une forme d'onde dont la période, l'amplitude et le rapport cyclique sont réglables.

7.  Pèse-personne selon l'une des revendications précédentes, **caractérisé en ce que** ledit générateur de tension (10) est destiné à fournir un signal en tension (11) qui est un signal basse tension d'amplitude comprise entre 3 et 6V.

8.  Pèse-personne selon l'une des revendications précédentes, **caractérisé en ce qu'**il comporte un deuxième module électronique (7) destiné à mesurer le poids de l'individu.

9.  Pèse-personne selon la revendication 8, **caractérisé en ce que** le premier module électronique (6) et le deuxième module électronique (7) sont reliés à une unité de calcul (14) apte à déterminer la composition corporelle de l'individu.

10. Pèse-personne selon la revendication 9, **caractérisé en ce que** l'unité de calcul (14) est un microprocesseur.

11. Pèse-personne selon l'une des revendications précédentes, **caractérisé en ce qu'**il comporte un moyen d'affichage (15) des valeurs mesurées et des valeurs calculées.

12. Pèse-personne selon l'une des revendications précédentes, **caractérisé en ce qu'**il comporte au moins deux électrodes d'excitation (20,22) destinées à appliquer ledit signal électrique (11) entre un premier point et un deuxième point sur le corps de l'individu et au moins deux électrodes de mesure (24,26) aux bornes desquelles est mesurée une tension.

**Claims**

1.  A weighing scale comprising an apparatus for measuring the body composition of an individual having a first electronic module (6) intended to measure a bioelectric impedance and at least one electrical energy source intended to generate a variable electrical signal that passes through the body or a part of the body of the individual when this latter is connected to the measuring apparatus by means of excitation electrodes, **characterised in that** said energy source is a voltage generator (10) intended to furnish a voltage signal (11) of rectangular waveform, which is applied to the terminals of the excitation electrodes of the apparatus, and **in that** said first electronic module (6) measures the voltage at the terminals of the measuring electrodes of the apparatus.

2. A weighing scale according to claim 1, **characterised in that** the calculation means of the apparatus determine the equivalent resistance depending on the extracellular and intracellular resistances of the body or of the measured part of the body of the individual .

3. A weighing scale according to claim 2, **characterised in that** said first module (6) comprises means to measure voltages $V_b$ and $V_c$ at the terminals of the measuring electrodes of the apparatus and voltages $V_r$ and $V_d$ at the terminals of a reference resistance $R_r$ and **in that** it comprises means to calculate the equivalent resistance of the body or of the measured part of the body of the individual according to the formula: $R_{eq}=R_r*(V_b-V_c)/(V_d-V_r)$.

4. A weighing scale according to claim 3, **characterised in that** said measuring means carry out voltage measurements at least two predetermined moments of the duration of said signal (11) and calculation means in order to directly determine the equivalent resistance $R_{eq}$ of the body or of the measured part of the body of the individual at these two moments and to deduce therefrom its extracellular resistance $R_e$ and its intracellular resistance $R_i$.

5. A weighing scale according to claim 4, **characterised in that** it comprises means for carrying out a first voltage measurement , at the end of a plateau (32) of said signal (11) of rectangular waveform, in order to determine the equivalent resistance $R_{eq}$, equal to the extracellular resistance $R_e$, as well as a second voltage measurement, at the end of a rising edge (31) of said signal (11) to determine the equivalent resistance $R_{eq}$ equal to $R_e$ in parallel with $R_i$ and to determine, on the basis of the first measurement, the intracellular resistance $R_i$ of the body or of the measured part of the body of the individual.

6. A weighing scale according to one of the preceding claims, **characterised in that** said voltage generator (10) is intended to furnish a voltage signal (11) that presents a waveform whose period, amplitude and duty cycle are adjustable.

7. A weighing scale according to one of the preceding claims, **characterised in that** said voltage generator (10) is intended to furnish a voltage signal (11) that is a low voltage signal with an amplitude comprised between 3 and 6V.

8. A weighing scale according to one of the preceding claims, **characterised in that** it has a second electronic module (7) intended to measure the weight of the individual.

9. A weighing scale according to claim 8, **characterised in that** the first electronic module (6) and the second electronic module (7) are connected to a calculation unit (14) able to determine the body composition of the individual.

10. A weighing scale according to claim 9, **characterised in that** the calculation unit (14) is a microprocessor.

11. A weighing scale according to one of the preceding claims, **characterised in that** it has a display means (15) for the measured values and the calculated values.

12. A weighing scale according to one of the preceding claims, **characterised in that** it has at least two excitation electrodes (20, 22) intended to apply said electric signal (11) between a first point and a second point on the body of the individual and at least two measurement electrodes (24, 26) at the terminals of which a voltage is measured.

**Patentansprüche**

1. Personenwaage, umfassend ein Gerät zur Messung der körperlichen Zusammensetzung eines Individuums, umfassend ein erstes elektronisches Modul (6), das dazu bestimmt ist, eine bioelektrische Impedanz zu messen, und zumindest eine elektrische Energiequelle, die dazu bestimmt ist, ein variables elektrisches Signal zu liefern, das den Körper oder einen Teil des Körpers des Individuums durchströmt, wenn dieser mittels Erregungselektroden mit dem Messgerät verbunden ist, **dadurch gekennzeichnet, dass** die Energiequelle ein Spannungsgenerator (10) ist, der dazu bestimmt ist, ein rechteckiges Spannungssignal (11) zu liefern, das an den Anschlüssen der Erregungselektroden des Gerätes angelegt wird, und dass das elektrische Modul (6) die Spannungen an den Anschlüssen der Messelektroden des Gerätes misst.

**2.** Personenwaage nach Anspruch 1, **dadurch gekennzeichnet, dass** die Berechnungsmittel des Gerätes den entsprechenden Widerstand in Abhängigkeit von extrazellulären oder intrazellulären Widerständen des Körpers oder des gemessenen Körperteils des Individuums bestimmen.

**3.** Personenwaage nach Anspruch 2, **dadurch gekennzeichnet, dass** das erste Modul (6) Mittel zur Messung der Spannungen $V_b$ und $V_c$ an den Anschlüssen der Messelektroden des Gerätes und der Spannungen $V_r$ und $V_d$ an den Anschlüssen eines Referenzwiderstandes $R_r$ umfasst, und dass es Mittel zur Berechnung des entsprechenden Widerstands des Körpers oder des gemessenen Körperteils des Individuums nach der Formel: $R_{eq} = R_r{}^* (V_b - V_c) / (V_d - V_r)$ umfasst.

**4.** Personenwaage nach Anspruch 3, **dadurch gekennzeichnet, dass** die Mittel zur Messung an zumindest zwei vorausbestimmten Momenten der Dauer des Signals (11) Spannungsmessungen durchführen, und dass die Mittel zur Berechnung imstande sind, an diesen beiden Momenten direkt den entsprechenden Widerstand $R_{eq}$ des Körpers oder des gemessenen Körperteils des Individuums zu bestimmen und daraus seinen extrazellulären Widerstand $R_e$ und seinen intrazellulären Widerstand $R_i$ abzuleiten.

**5.** Personenwaage nach Anspruch 4, **dadurch gekennzeichnet, dass** sie Mittel umfasst, die die Durchführung einer ersten Spannungsmessung am Ende einer Phase (32) des rechteckigen Signals (11) ermöglichen, um den entsprechenden Widerstand $R_{eq}$ zu bestimmen, der gleich ist dem extrazellulären Widerstand $R_e$, sowie eine zweite Spannungsmessung am Ende einer ansteigenden Flanke (31) des Signals (11), um den entsprechenden Widerstand $R_{eq}$ zu bestimmen, der gleich $R_e$ ist, parallel zu $R_i$, und um auf Basis der ersten Messung den intrazellulären Widerstand $R_i$ des Körpers oder des gemessenen Körperteils des Individuums zu bestimmen.

**6.** Personenwaage nach einem der vorhergehenden Ansprüche, **dadurch gekennzeichnet, dass** der Spannungsgenerator (10) dazu bestimmt ist, ein Spannungssignal (11) zu liefern, das eine Wellenform aufweist, deren Periodendauer, Amplitude und Tastgrad regelbar sind.

**7.** Personenwaage nach einem der vorhergehenden Ansprüche, **dadurch gekennzeichnet, dass** der Spannungsgenerator (10) dazu bestimmt ist, ein Spannungssignal (11) zu liefern, das ein Niederspannungssignal ist, dessen Schwingungsweite zwischen 3 und 6 V beträgt.

**8.** Personenwaage nach einem der vorhergehenden Ansprüche, **dadurch gekennzeichnet, dass** sie ein zweites elektronisches Modul (7) umfasst, das dazu bestimmt ist, das Gewicht des Individuums zu messen.

**9.** Personenwaage nach Anspruch 8, **dadurch gekennzeichnet, dass** das erste elektronische Modul (6) und das zweite elektronische Modul (7) mit einer Berechnungseinheit (14) verbunden sind, die imstande ist, die körperliche Zusammensetzung des Individuums zu bestimmen.

**10.** Personenwaage nach Anspruch 9, **dadurch gekennzeichnet, dass** die Berechnungseinheit (14) ein Mikroprozessor ist.

**11.** Personenwaage nach einem der vorhergehenden Ansprüche, **dadurch gekennzeichnet, dass** sie ein Mittel zur Anzeige (15) der gemessenen und berechneten Werte umfasst.

**12.** Personenwaage nach einem der vorhergehenden Ansprüche, **dadurch gekennzeichnet, dass** sie mindestens zwei Erregungselektroden (20, 22), die dazu bestimmt sind, das elektrische Signal (11) zwischen einem ersten Punkt und einem zweiten Punkt am Körper des Individuums anzulegen, und zumindest zwei Messelektroden (24, 26) umfasst, an deren Anschlüssen eine Spannung gemessen wird.

EP 1 351 603 B1

Fig.1

Fig.2

Fig.3

10

Fig. 4

Fig. 5

Fig. 6

## RÉFÉRENCES CITÉES DANS LA DESCRIPTION

**Documents brevets cités dans la description**

- FR 2698779 A
- WO 9837829 A
- EP 0940120 A
- FR 2775581 A

**Littérature non-brevet citée dans la description**

- Thèse de Doctorat de Biologie Humaine. Université René Descartes, 1994, 85-86